Europäisches Patentamt

European Patent Office

Office européen des brevets

(11) Publication number: **0 287 361 B1**

# EUROPEAN PATENT SPECIFICATION

(45) Date of publication of patent specification: **29.06.94**   (51) Int. Cl.⁵: **A61K  39/395**, A61K 39/35

(21) Application number: **88303350.8**

(22) Date of filing: **14.04.88**

(54) **Treatment of allergy and composition therefor.**

(30) Priority: **16.04.87 US 38985**

(43) Date of publication of application:
**19.10.88 Bulletin  88/42**

(45) Publication of the grant of the patent:
**29.06.94 Bulletin  94/26**

(84) Designated Contracting States:
**DE FR GB**

(56) References cited:
**EP-A- 0 152 270**
**EP-A- 0 178 085**
**EP-A- 0 200 412**
**US-A- 4 564 600**

(73) Proprietor: **INTERNATIONAL INSTITUTE OF CELLULAR AND MOLECULAR PATHOLOGY (ICP)**
**Avenue Hippocrate 75**
**B-1200 Brussels(BE)**

(72) Inventor: **Saint-Remy, Jean-Marie**
**79 rue du Lambais**
**B-5980 Grez-Doiceau(BE)**
Inventor: **Lebeque, Serge**
**55 rue Edouard Speekaert**
**B-1200 Brussels(BE)**
Inventor: **Lebrun, Philippe**
**31 rue Alfred Bequet**
**B-5000 Namur(BE)**
Inventor: **Masson, Pierre Lucien**
**Avenue Emile Vandervelde 107**
**B-1200 Brussels(BE)**

(74) Representative: **Bassett, Richard Simon et al**
**ERIC POTTER & CLARKSON**
**St. Mary's Court**
**St. Mary's Gate**
**Nottingham NG1 1LE (GB)**

**Description**

Background of the Invention

This invention relates to a method of treating allergy, particularly immediate hypersensitivity, and to pharmaceutical compositions useful therefor.

Immediate hypersensitivity, that is, anaphylactic response, is a form of allergic reaction which develops very quickly, i.e., within seconds or minutes of exposure of the patient to the causative allergen and which is mediated by IgE antibodies made by B lymphocytes. There is very little IgE in non-allergic patients, but in a person suffering from allergy, the concentration of IgE is very much higher. This elevated amount of IgE mediates immediate hypersensitivity by priming mast cells which are abundant in the skin, lymphoid organs, membranes of the eye, nose and mouth, and respiratory tree and intestines. Mast cells have surface IgE receptors, and the elevated concentrations of IgE in allergy-suffering patients become bound to them. When this bound IgE is subsequently contacted by the appropriate allergen, the mast cell is caused to degranulate and release various substances such as histamine into the surrounding tissue. It is the release of these substances which is responsible for the clinical symptoms typical of immediate hypersensitivity, namely, contraction of smooth muscle in the airways or the intestine, the dilation of small blood vessels and increase in their permeability to water and plasma proteins, secretion of thick, sticky mucus and, in the skin, the stimulation of nerve endings that results in itching or pain.

Immediate hypersensitivity is, at best, a nuisance to the sufferer. At worst it can present very serious problems and can in rare, extreme cases result in death. Efforts have been made for many years to find some way of effectively treating sufferers, and essentially three such ways have been found. These are: avoidance of the allergen, desensitization and the use of drugs. Of these, avoidance of the allergen is in one sense clearly the best approach, but of course, it is in practice very difficult, and usually impossible, to achieve. Treatment by the use of drugs is useful, but it is generally directed to alleviating the symptoms of allergy rather than dealing with its causes. Also, there are disadvantages in the use of certain drugs, and it is by no means always possible with drugs to assist patients to the extent desired.

The third method of treatment, namely, desensitization, has long been recognized as perhaps the most hopeful practical approach to the problem. It has been known for over 60 years that the injection into a patient of initially small but subsequently increasing amounts of the offending allergen itself, over a period of time, can often result in an improved resistance to that allergen. This procedure is known as "desensitization" or "allergen immunotherapy". While it is not useful in the treatment of food allergies, it is useful in the treatment of, for example, inhalant allergen-derived sensitivity and allergic reactions due to insect stings.

Curiously, although desensitization has been successfully practiced for many years, the mechanism by which it works is still not known. In most patients, the injection of the allergen appears to give rise, not to IgE antibodies, but rather to IgG antibodies which, upon inhalation by the patient of the allergen in question, combine with the allergen to block its ability to bind to the mast cell IgE. These IgG antibodies are called "blocking antibodies". However, this hypothesis does not always fit the facts. In some patients who are successfully desensitized, there is little specific IgG in their blood, and in other patients for whom the injections have apparently been ineffective against allergy, there is a large amount of the particular IgG.

Whatever the mechanism may be, it remains the fact that many though not all sufferers of immediate hypersensitivity can be helped considerably by desensitization. The technique involves injecting the allergen to which the patient has become sensitized into the patient over a relatively long period of time, e.g., one year or more. Initially, the doses used are very small, but in the absence of contra-indications, they are increased rapidly to high levels which are necessary if the treatment is to be effective.

There are certain problems in desensitization treatment. First, it is necessary for the patient to have injections very frequently, e.g., initially every two or three days, gradually reducing to once every two or three weeks. This is not only a time-consuming procedure, but is also disruptive of the patient's normal routine, and generally undesirable. Also, the dose of allergen administered has to be carefully monitored and controlled, which adds to the complexity of the procedure. A second problem is that, in the treatment itself, there is an element of risk to the patient. Even though initial doses of the allergen are very small and precautions are routinely taken to watch for any allergic response, local or systemic allergic reactions such as hives, asthma and fainting do sometimes occur and can cause death in exceptional cases. For these and other reasons, many practicing physicians are skeptical of desensitization techniques.

Attempts have been made in the past to overcome or avoid these problems. To reduce the frequency of injection, preparations have been administered which release the allergen slowly over a period of time. These have not been very successful for a number of reasons, an important one of which being that, once administered, no control can be exercised over the amount of allergen released into the patient's blood. Another way in which the frequency of injection might be reduced would be to devise a treatment whereby the necessary large doses of allergen are administered rather earlier to the patient, but to date there has been no such treatment devised. Attention is also being directed to the possibility of administering modified allergens instead of the "pure" material itself. Thus, attempts have been made to modify an allergen chemically so that, while its immunogenicity, i.e., its ability to cause an immune response in the patient, is unchanged, its allergenicity is substantially reduced. Success has been achieved with this approach, but it has certain disadvantages of its own. First, each allergen, and there are of course a vast number of allergens against which patients can become sensitized and thus need desensitization treatment, has to be modified individually in accordance with its particular chemical structure, such that there is no satisfactory, universally applicable technique for modifying allergens for a desensitization treatment. Second, a very considerable amount of work can be involved in devising an acceptable modified allergen, bearing in mind the requirements for it to be useful in the desensitization treatment, including the necessity for the chemical modification itself not to cause any adverse reaction in the patient. Third, because accurate control of dose is so important in a desensitization treatment, there can be problems with modified allergens in determining the proper dose required.

Further background information on allergy and desensitization treatments may be found in Paul D. Buisseret, "Allergy", Scientific American, August 1982, pp. 82-91; Howard J. Sanders, "Allergy: A Protective Mechanism Out of Control", C & E News, vol 48, pp. 84-134 (1970); and "Primer on Allergic and Immunologic Disease", Journal of the American Medical Association, volume 248, no. 20 (November 26, 1982).

Summary of the Invention

There is provided a pharmaceutical composition for administration to human beings, for the treatment of immediate hypersensitivity to an allergen, said composition comprising an immune complex of an allergen and a purified antibody specific thereto, said allergen being selected from a specific subclass of antigen that can cause immediate hypersensitivity that is mediated by IgE antibody, said allergen also comprising an in vitro synthesised antigenic determinant coupled to a carrier protein; said antibody being present in at least a molar equivalent of antigenic determinant; and a pharmacologically acceptable carrier or diluent.

There is also provided a pharmaceutical composition for administration to human beings, for the treatment of immediate hypersensitivity to an allergen, said composition comprising an immune complex of an allergen and a purified antibody specific thereto, said allergen being selected from a specific subclass of antigen that can cause immediate hypersensitivity that is mediated by IgE antibody and said allergen also being chemically modified by glutaraldehyde or ethylene glycol derivatives; said antibody being present in at least a molar equivalent of the allergenic determinants of said allergen; and a pharmacologically acceptable carrier or diluent.

There is further provided the use of a mixture of an allergen and an antibody specific thereto in the manufacture of a medicament for reducing the immediate hypersensitivity of a human being to the allergen, said allergen being selected from a specific subclass of antigen which can cause immediate hypersensitivity which is mediated by IgE antibody and said allergen also comprising an in vitro synthesized antigenic determinant coupled to a carrier protein, said antibody being present in at least a molar equivalent of antigenic determinant.

There is yet further provided the use of a mixture of an allergen and an antibody specific thereto in the manufacture of a medicament for reducing the immediate hypersensitivity of a human being to the allergen, said allergen being selected from a specific subclass of antigen which can cause immediate hypersensitivity which is mediated by IgE antibody and said allergen also being chemically modified by glutaraldehyde or ethylene glycol derivatives, and wherein said antibody is present in at least molar equivalent of the allergenic determinants of said allergen.

As disclosed in EP-A-0178085, by mixing the allergen with an antibody thereto, a number of advantages are achieved over other prior art procedures. First, this is a universally applicable and relatively simple way of treating an immediate hypersensitivity. Antibody is raised to any and every allergen, and the mere admixture thereof with the allergen is a straightforward procedure. Second, when the antibody is derived from the patient, the risk of any adverse reaction thereto is virtually eliminated. This is in sharp contrast to prior art procedures for chemically modifying allergens, where adverse reactions are sometimes obtained. Indeed, where an allergen is modified by coupling it chemically to another material, the patient can become sensitized to the other material. This does not occur with the use of naturally occurring antibody. Alternatively, the antibody is derived from pooled human gammaglobulins or monoclonal antibodies.

The clinical efficacy of the present invention is very good. Patients who have failed to respond to prior art desensitization techniques have been found to benefit from treatment with the present invention. The duration of treatment of the present invention is in certain embodiments markedly less than in prior known procedures. Further, it is usually possible in accordance with the present invention to reach much higher allergen doses more quickly than in prior known procedures, although this is not necessary in order for the clinician to administer large quantities to initiate an efficient response. Also, the treatment with the invention does not appear to generate any significant local or systemic allergic reaction, and thus the risks in its use are less than those using prior art modified allergens.

The mechanism by which such treatment works is not known. It is hypothetical that the mixture of allergen and antibody inevitably contains allergen: antibody immune complex, and that the antibody thus masks any allergenicity of the allergen. This would explain the very much reduced allergenicity of the complex. The reason why treatment with the complex can achieve such a marked desensitization is not understood, and indeed any such explanation may have to wait for a better understanding of the mechanism of desensitization generally. It is surprising that mixtures of allergens and antibodies thereto are so effective as desensitizing agents, in that there is no apparent reason why they should be. Regardless, the scope of the ensuing claims is not intended to be limited in any way by a specific actual or hypothesized mechanism of action.

It is not uncommon for patients who suffer anaphylactic response to a particular allergen, also to suffer such a response to one or more other allergens. However, it is possible to desensitize such a patient in respect of two or more allergens simultaneously, by administering the said allergens in admixture with antibodies against each allergen. Thus, in one embodiment, a composition comprises a mixture of two allergens and two families of antibodies, each family directed against a respective allergen. Alternatively, simultaneous desensitization in respect of two or more allergens can be effected by administering two or more compositions of the invention, each composition comprising one allergen only and its respective antibodies.

We have referred above to the compositions comprising antibodies against the allergen. It should be understood that, instead of whole antibody, antibody fragment such as F(ab')2 is used in an alternative embodiment. In a still further embodiment, monoclonal antibodies are used.

In treating a patient for allergy, there are essentially three steps, namely:

(1) Identification of the allergen and preparation of the antibody thereto;

(2) Formation of the mixture of allergen and antibody to make a treatment composition and

(3) Administration of the composition to the patient.

These steps will now be described in more detail.

## 1. Identification of the Allergen and Preparation of the Antibody

The allergen responsible for an allergy can be determined by standard known techniques. An antibody thereto is then generated. Three sources of antibody can be used: (a) immunized animals, (b) individual blood donors and pooled plasma from multiple donors, and (c) the patient himself. It is preferred to use antibodies from the patient because the patient will normally have larger amounts of the specific antibodies required than will blood donors. On the other hand, the use of antibodies from pooled plasma is commercially desirable since it allows the preparation of pre-packaged allergen-antibody complexes without involving the patient. Antibodies of animal origin are generally the least desirable to use because of the risk of undesirable side reactions.

The antibody is suitably polyclonal or monoclonal and is used, for example, in a purified state or in the form of an immunoglobulin fraction. Purification of the antibody has the advantage of removing therapeutically irrelevant materials. The use of polyclonal antibodies decreases the risk of allergenic reactions against unmasked antigenic determinants.

The antibody is suitably purified by various known techniques such as, for example, specific absorption on the allergen which has been insolubilized by coupling to a solid phase. The antibody is then recovered by elution under conditions which dissociate the allergen-antibody complex, such as conditions of extreme pH, or by the use of chaotropic agents.

## 2. Formation of the Composition

Compositions are made by mixing the allergen or allergens with the antibody or antibodies, in a form suited to the particular mode of administration that is selected. Because the antibody will react only with its specific allergen, almost any preparation of allergen, even in the form of crude extracts, is suitably used, provided it is devoid of toxic substances. However, the use of pure or relatively pure preparations of allergen is preferred because it is then easier to assess and control the amount of allergen present, which is important in controlling doses.

In one embodiment, the allergen is chemically modified by glutaraldehyde, ethylene glycol derivatives or the like. In another embodiment, the allergen comprises an in vitro synthesised antigenic determinant coupled to a carrier protein.

The proportion of antibody to be added to the allergen is defined essentially by the neutralizing power of the antibody. The ratio of allergen to antibody depends largely on the size of the allergen, since the number of antigenic determinants on the allergen is in general proportional to the molecular weight of the allergen. Enough antibody must be used so that, when the composition is administered, there is practically no allergic effect by the allergen. The minimum amount of antibody is normally a molar equivalent of allergen, and the antibody is preferably present in a molar excess relative to the amount of allergen. If desired, routine testing reveals for any particular allergen and antibody, the minimum amount of antibody to be used. There is no maximum to the amount of antibody which is used. For safety, a molar excess of up to about 500 is used. An even larger antibody excess can be utilized but it is, of course, wasteful of the valuable material. A suitable dosage range for the allergen is from about 1 ng to about 100 μg. The strength of the compositions is preferably expressed in terms of the allergen concentration and the allergen to antibody ratio. The selection of suitable ratios of allergen to antibody and allergen dosages is illustrated in the foregoing examples.

One simple method of preparing the mixture of allergen and antibody, which avoids the necessity of purifying the allergen or the antibody, is the use of the immune precipitate. In one embodiment, the precipitate is prepared by incubating a crude preparation of immunoglobulin from the patient's plasma or serum with the allergen, and then centrifuging. The precipitation process is enhanced by the addition of polymers such as polyethylene glycol and dextran, or biological reagents such as rheumatoid factor or the Clq factor of complement. These techniques are well known.

The compositions of the invention contain, in addition to the allergen and antibody, any other suitable components. For example, in the case of injectables, suitable additional components include human albumin to prevent denaturation of antibody, antiseptic agents such as phenol and the like, and adjuvants such as peptidoglycans, tyrosine crystals and the like.

Suitable liquid carriers for the composition when in injectable form include distilled water, or more preferably saline or buffered saline. In preferred embodiments, a saline carrier of 9 grams per liter of sodium chloride is used, or a buffered saline carrier having a pH of 7.4. In general, suitable liquid carriers are of low irritance, e.g., of neutral pH and physiological ionic strength. The selection of other pharmacologically acceptable carriers and diluents for use in the compositions is within the skill of those ordinarily skilled in the art.

The compositions of the invention are prepared in a variety of forms depending on the manner of administration. Thus, they are suitably prepared in a sterile injectable form, slow-release implant form, a form suitable for local application to nasal, bronchial, lacrimal and/or gastrointestinal mucosae, in which case they are suitably in an aerosol or spray form or in a form similar to eye or nose drops, or as protected enteric capsules or the like. Other suitable forms will be readily apparent to those ordinarily skilled in the art.

When the compositions are prepared in liquid form, the liquids are suitably solutions or suspensions. The liquids are suitably stored in ampoules or are lyophilized and reconstituted immediately prior to use. The compositions of the invention are fairly stable and, in sterile ampoules, can normally be stored at 4 °C for a limited time, or at -20 °C for 12 to 24 months. When lyophilized, their storage life is much longer.

3. Administration of the Compositions

The injectable compositions are suitably injected in various ways: intradermally, subcutaneously, intramuscularly and, with great care, intravenously. The intradermal route is preferred in that it has the advantage of causing a very clear skin reaction if insufficient antibody is present to provide the necessary neutralizing of the allergen in the composition. The frequency of injections varies very widely, for example from daily to yearly, depending upon the type of allergen, the severity of the disease and the stage of desensitization. The dose of allergen is usually doubled at each injection, which is, of course, a very fast increase in dose. Greater or lesser increases are of course suitable depending upon a particular clinical situation.

Compositions in forms other than injectables are administered in any suitable manner. For example, respiratory allergy is treated by aerosol administration using, for example, a Micronebulizer (Bird) to give

EP 0 287 361 B1

particles between 0.5 and 5 um at a rate of 0.35 ml/min, the patient being instructed to breathe in once deeply and to hold his or her breath for two seconds. The dose is repeated at, for example, weekly intervals over the desired period.

The quantity of allergen in the composition used for the first administration, e.g., injection, is suitably equal to and usually much higher, for example, eighty times higher, than those amounts of allergen used in classical desensitization techniques. Thus, in classical techniques, the usual initial dose is from about $10^{-10}$g to $10^{-9}$g, for example, from 0.1 to 1 nanogram of purified allergen from the house dust mite is used for the first injection in classical desensitization techniques. However, a suitable initial dose of allergen is used in an allergen:antibody molar ratio of 1:1 to 1:500, and in the preferred embodiment the amount of antibody exceeds that of allergen, in an allergen:antibody molar ratio of at least 3:500. The preferred initial dose is normally different from one patient to another and corresponds to that dose which causes a skin reaction of 3 to 5 mm diameter after intradermal injection.

A solid phase support for the allergen in alternative embodiments includes membranes and the like.

Comprehensive listings of allergens that can trigger immediate hypersensitivity which is mediated by IgE antibody are set forth in proposed rules of the Federal Food and Drug Administration, reported at Federal Register, Vol. 50, No. 15, pages 3082-3288 (January 23, 1985) and in a notice issued by that agency, reported at Federal Register, Vol. 50, No. 154, pages 32314-32318 (August 9, 1985). Compositions include complexes of the allergens listed in those publications with the corresponding specific antibodies. In order that the invention may be more fully understood, the following examples illustrate the treatment of patients suffering from hypersensitivities to representative allergens.

Prior art EXAMPLE 1

Treatment of Patients Suffering from Allergic Asthma Caused by House Dust Mite (Dermatophagoides pteronyssinus or DPT)

1. Patients

The three patients of Example 1 are identified as follows:

| Patient | Initials | Sex | Age |
|---------|----------|-----|-----|
| 1 | L.L. | F | 40 |
| 2 | B.J. | M | 21 |
| 3 | W.E. | F | 37 |

The patients were selected on the following criteria:
1. Long-term (more than 5 years) history of invalidating bronchial asthma, keeping them away from work at least 3 months a year and having necessitated at least one admission in intensive care.
2. Evidence for an extrinsic asthma where DPT was clearly the causative agent.
3. High sensitivity to DPT shown by intradermal testing and bronchial provocation test with the allergen.
4. High level of DPT-specific antibodies.
5. A history of at least one unsuccessful classical desensitization to DPT.
6. No permanent corticotherapy.

All three patients were taking drugs daily. These included theophylline and derivatives, $\beta_2$ agonists in aerosol and topical nasal beclomethasone in one patient (B.J.). They were not suffering from any other known disease, except that two of them (B.J. and W.E.) had a chronic rhinitis. One patient (W.E.) was also highly sensitive to grass pollen as shown by hay fever and asthma during the pollen season and by a high level of specific IgE antibodies against pollens.

The patients were submitted to a 3-month treatment, using antigen-antibody complexes made with their own purified antibodies not according to the present invention.

## 2. Antibody Purification

### a. Plasma collection and handling

One hundred ml of plasma from each patient were precipitated with 18% $Na_2SO_4$ at 37°C. for 4 hours. The precipitate was washed and resuspended in phosphate buffered saline (PBS) containing 1M NaCl and, after centrifugation to clear off small particles, filtered through a 0.45 $\mu$ filter. Twenty-five ml of this solution were applied onto a 9 x 90 cm TSK HF-55 (Merck, Darmstadt) gel column, chromatographed at a rate of 250 ml/h and recovered in 10 ml fractions. The two main peaks represented IgM and IgG (plus IgA and IgE). Cross contamination was about 5% as shown by immunodiffusion.

IgM and IgG (plus IgA and IgE) were concentrated separately by ultrafiltration through an XM-100 Amicon membrane to a volume of ~25 ml and dialyzed for 3 days against PBS with several changes of the dialysis bath. The solutions were then passed through a 0.22 $\mu$ GV filter (Millipore) and stored in sterile conditions.

### b. Preparation of the Immunoadsorbent

Commercially available allergens were purified by gel filtration chromatography on Ultrogel AcA 44 and/or Ultrogel AcA 54 (LKB) and, in some cases, by specific immunoadsorption on insolubilized polyclonal or monoclonal specific antibodies.

The allergen was then coupled with carbodiimide to carboxylated agarose (CH-Sepharose 4B; Pharmacia Fine Chemicals). For this purpose, the allergen was incubated at pH 4.5 with 0.1M carbodiimide and carboxylated agarose for 24 hr. at 21°C.

The remaining reactive groups on the solid phase were inactivated by its incubation with 1M glycine for 3h. at 21°C. The immunoadsorbent was then washed alternatively with 0.1M acetate buffer pH 4.0 and 0.1M carbonate buffer pH 8.3, both containing 0.5M NaCl. To avoid the elution of undesired material with the antibodies of interest, the gel was submitted prior to immunoadsorption to the elution conditions to be described hereafter and to an additional washing with 3M ammonium thiocyanate.

### c. Extraction of the Specific Antibodies

The immunoglobulin fractions (1-2 g) from each of the patients were applied onto an immunoadsorbent column (5 ml; 10 x 2 cm; flow rate 20 ml/h) and the specific antibodies recovered after appropriate washings. Washing was accomplished by:

1. Washing with PBS until the optical density at 280 mm was less than 0.02.
2. Washing with PBS containing 1M NaCl to eliminate non-specific adsorption.
3. Washing with 50 ml of 9 g/l NaCl.
4. Eluting with successive aliquots of 50 ml citric acid, pH 2.7 followed by PBS.

Each new wash and elution step was pursued until no protein was detectable in the effluent. Fractions eluted with citric acid and PBS were pooled immediately, neutralized with dropwise addition of 2M TRIS-HCl buffer, concentrated on a YM 10 ultrafiltration membrane and dialyzed against PBS for 48 h. The eluate was then filtered through a 0.22 $\mu$ filter and stored at 4°C. in sterile vials. The immunoadsorbent was washed with 3M ammonium thiocyanate for 20 min and finally with 100 ml PBS. All buffers were filtered in 0.22 $\mu$ filter.

### d. Yield and Class Repartition of Specific Antibodies

The amounts of specific antibodies, estimated by optical absorbance of 280 nm, ranged from 2 to 6 mg per 100 ml of plasma.

The analysis of the eluted antibodies failed to reveal the presence of autoantibodies such as rheumatoid factor (anti-IgG autoantibody) and showed that the specific antibodies were of the following classes: IgG (50%), IgM (35%), IgA (14.5%) and IgE (0.5%). No other plasma protein was detected in significant amounts by immunonephelometry.

EP 0 287 361 B1

### 3. Preparation of Antigen-Antibody Complexes

#### a. Precipitation Curve

To determine the optimal ratio of allergen versus antibody at which most antigenic determinants are hidden by specific antibodies, we made a precipitation curve as follows. Into a series of tubes containing the same amounts of antibodies were pipetted increasing dilutions of allergen in 0.1M borate buffer pH 8.5. Polyethylene glycol was then added to a final concentration of 200 g/l. After incubation for 4 h. at 21°C., then for 16 h. at 4°C. and centrifugation at 8,000 g for 20 min., the precipitates were washed and the amount of protein in the supernatant and the precipitate estimated by the Lowry technique. For safety purposes, we used for the injection 1/5 of the amount of allergen giving the largest precipitate. In these conditions, the antibody was in large excess (allergen to antibody weight ratio = 1:500).

#### b. Preparation of the Complexes and Injectable Compositions

Allergen and antibody were mixed in a weight ratio of 1:500 in 9 g/l NaCl containing 0.3% human serum albumin and 0.4% phenol. All solutions were passed through a sterile 0.22 $\mu$ filter and handled in sterile conditions. The final volume was 2 ml and contained 400 $\mu$g antibody and 800 ng allergen. The injectable solutions were kept in sealed vials at 4°C. until use.

### 4. Injections

#### a. Patients' Tolerances

To assess the patients' tolerances to the compositions, we serially diluted the allergen in the presence of a constant amount of antibodies (the compositions being generally as described in paragraph 3(b) above). Each of these dilutions was then injected intradermally in 20 $\mu$l aliquots starting with the lowest allergen-antibody ratio. An interval of 15 minutes was then allowed between the injections. The highest allergen-antibody ratio giving an acceptable skin reaction (wheal of maximum 3 cm diameter) was chosen and used throughout the study. At a ratio of 1:500, the complex usually caused a small skin reaction or none at all.

#### b. Injection Scheme

Intradermal injections on the internal side of the arm were repeated every week for six weeks, then every fortnight for a total of three months. In a typical scheme, a volume of 20 $\mu$l containing 4 $\mu$g antibody and 8 ng allergen was used for the first injection. This volume was doubled every week up to a maximum of 200 $\mu$l and maintained to the end of the study (a total of 3 months).

### 5. Clinical Outcome

#### a. Subjective Assessment

No side effects were noted. The patients were reported to feel well and improved as far as their asthmatic symptoms were concerned. No one injection in any of the three patients gave a clear allergic reaction (there were 40 injections in all). At the injection site, there was either a relatively weak skin reaction or none at all.

#### b. Clinical Assessment

Three criteria were used to evaluate the clinical outcome of the patients: (1) skin reactivity to the allergen, (2) bronchial provocation test with the allergen, and (3) baseline lung function.

#### 1. Skin Reactivity

The allergen was serially diluted in 9 g/l NaCl with 0.3% albumin and 0.4% phenol, and 20 $\mu$l was injected intradermally into the arm. After 20 minutes, the wheal area was measured by planimetry and plotted on a graph against the allergen concentration. The amount of allergen needed to obtain a certain

8

wheal area was then read on the curve. The same preparation of allergen was used for the tests made before and after immunotherapy. In the three patients, it was found necessary after treatment to use 16 times more allergen to induce a skin reaction as intense as the one observed before treatment.

2. Bronchial Provocation Test

To assess the bronchial reactivity to DPT before and after immunotherapy, the patients were submitted to aerosols of DPT at different dilutions. Under well standardized conditions, the forced expiratory volume per second ($FEV_1$) and airway conductance were assessed. By plotting the values of these two parameters against the allergen dilution, the dilution of allergen giving a 20% fall in $FEV_1$ or a 35% decrease in airway conductance was determined. Non-specific bronchial reaction to acetylcholine was assessed in this way. Table 1 compares the bronchial sensitivity of patients 1 to 3 before and after three months of immunotherapy.

## TABLE 1A

### Non-Specific Bronchial Reactivity to Dilute Acetylcholine and Specific Bronchial Reactivity to Dilute DPT Before and After Three Months of Immunotherapy

| Patient | Reactivity to acetyl- choline | | Reactivity to DPT | |
|---|---|---|---|---|
| | Before | After | Before | After |
| 1. (L.L.) | $10^{-3}$ | ND* | $10^{-3.8}$ | ND |
| 2. (B.J.) | $10^{-2.5}$ | $>10^{-2}$** | $10^{-2.6}$ | $>10^{-1}$** |
| 3. (W.E.) | $10^{-3}$ | $10^{-3.5}$ | $10^{-4}$ | $>10^{-1}$** |

*ND means "not determined".

**No bronchial reactivity observed at the highest concentration of acetylcholine or DPT used.

3. Baseline Lung Function

Baseline $FEV_1$ and airway resistance were assessed during the clinical follow-up. The three patients of Example 1 achieved over 100% of the normal values (111, 127 and 102% respectively for patients 1, 2 and 3) after three to four weeks of treatment. These values were maintained throughout the study except for patient 1 where the $FEV_1$ dropped to 70% of the normal; for this reason no bronchial provocation test was done for this patient.

6. Laboratory Investigations

a. Specific Antibodies

Laboratory investigations of DPT-specific antibodies were made before and after 9 weeks of treatment. Results are given in Table 1B.

### TABLE 1B
#### DPT-Specific Antibodies Before and
#### After Nine Weeks of Treatment

| Patient | IgG* | | IgE** | | Total IgE | |
|---|---|---|---|---|---|---|
| | Before | After | Before | After | Before | After |
| 1. (L.L.) | 31 | 45 | 16 | 124 | 1,696 | 1,470 |
| 2. (B.J.) | 31 | 34 | 118 | 288 | 2,182 | 2,058 |
| 3. (W.E.) | 27 | 32 | 10 | 42 | 855 | 551 |

\*in $\mu$g/ml

\*\*in ng/ml

It can be seen from Table 1B that (a) DPT-specific IgG increased moderately, (b) DPT-specific IgE increased dramatically, and (c) total IgE tends to decrease slightly. The same profile was observed for the three patients.

b. IgE Synthesis in Vitro

The total amount of IgE synthesis in vitro was evaluated on peripheral lymphocytes maintained in culture for 7 days. IgE was assayed in the supernatant by radioimmunoassay. The results are shown in Table 1C.

## TABLE 1C

### Total IgE Synthesis

| Patient | Weeks | | | | | |
|---|---|---|---|---|---|---|
| | 1 | 3 | 7 | 11 | 15 | 18 |
| 1. (L.L.) | 1,300* | 850 | 395 | 1,500 | 380 | 7,400 |
| 2. (B.J.) | 1,600 | 810 | 400 | 200 | ND** | <50 |
| 3. (W.E.) | 6,250 | 4,500 | 300 | 350 | ND | <50 |

*in pg/ml supernatant.

**ND means "not determined".

Results indicated in Table 1C show that in vitro production of total IgE tends to decrease in all three patients through the seventh week of the study. These results continue in Patients 2 and 3 through the 18th week. Total IgE levels, however, are influenced by several factors. During the course of the study it was determined that Patient 1 had developed an infectious bronchitis which may account for the increase in the total IgE count.

Prior art EXAMPLE 2

Treatment of DPT Hypersensitive Patients - First Dosage Scheme

Twenty-six patients were selected who were hypersensitive to Dermatophagoides pteronyssinus (DPT) and were suffering from severe bronchial asthma, wheezing and shortness of breath, causing occasional interruption of normal activities. For years, these patients took oral corticosteroids for short periods of time in addition to the regular use of theophillyne derivatives, beta-2-agonists and sodium cromoglycate.

Thirteen patients, randomly selected, were treated by fortnightly intradermal injections of a solution containing the allergen not according to the present invention with their own purified allergen-specific antibodies. The composition was prepared as described in Example 1. The patients received DPT allergen and their own purified allergen-specific antibodies at a weight ratio of 1:5. The mixture was initially made of 40 ng of antibody and 8 ng of allergen. The amount of injected material was increased according to the injection scheme listed in Table 4 up to the maximal dose of 3200 ng of antibodies and 640 ng of allergen. The treatment, which started in November 1985, lasted one year, with a maintenance dose, made of 3200 ng of antibody and 640 ng of allergen, being since November 1986 injected every six weeks. Thirteen patients served the as control group.

No systemic or side effects were noted. Although the patients of the control group experienced heavy symptoms with the need for corticosteroids intake during the year, the 13 treated patients had significant reduction of their clinical symptoms, reduction in medication intake and no need for corticosteroids.

TABLE 4

| Injection Scheme for DPT Hypersensitive Patients | | |
|---|---|---|
| Injection | Antibody(ng) | Allergen(ng) |
| 1 | 40 | 8 |
| 2 | 80 | 16 |
| 3 | 160 | 32 |
| 4 | 200 | 40 |
| 5 | 400 | 80 |
| 6 | 400 | 80 |
| 7 | 400 | 80 |
| 8 | 800 | 120 |
| 9 | 600 | 160 |
| 10 | 1200 | 240 |
| 11 | 1600 | 320 |
| 12 | 2000 | 400 |
| 13 | 2400 | 480 |
| 14 | 2800 | 560 |
| 15 | 3200 | 640 |
| " | " | " |
| " | " | " |
| 26 | 3200 | 640 |

Prior art EXAMPLE 3

Treatment of DPT Hypersensitive Patients - Second Dosage Scheme

Thirteen patients were selected who were hypersensitive to Dermatophagoides pteronyssinus (DPT) and who were suffering from severe bronchial asthma, wheezing and shortness of breath, causing occasional interruption of normal activities. For years, these patients took oral corticosteroids for short periods of time in addition to the regular use of theophillyne derivatives, beta-2-agonists and sodium cromoglycate.

These thirteen patients were treated by fortnightly intradermal injections of a solution containing the allergen not according to the present invention with their own purified allergen-specific antibodies. The composition was prepared as described in Example 1. The patients received DPT allergen and their own purified allergen-specific antibodies in a weight ratio of 1:5. The mixture was initially made of 40 ng of antibody and 8 ng of allergen. The amount of injected material was increased according to the injection scheme listed in Table 5 up to the maximal dose of 400 ng of antibodies and 80 ng of allergen. The treatment, which started in November 1985, lasted one year, with a maintenance dose being since November 1986 injected every six (6) weeks, said dose comprising 400 ng of antibody and 80 ng of allergen. The thirteen patients from Example 4 above served as the control group.

No systemic or side effects were noted. Although the patients of the control group experienced heavy symptoms with the need for corticosteroids intake during the year, the 13 treated patients had significant reduction of their clinical symptoms, reduction in medication intake and no need for corticosteroids.

TABLE 5

| Injection Scheme for DPT Hypersensitive Patients | | |
|---|---|---|
| Injection | Antibody(ng) | Allergen(ng) |
| 1 | 40 | 8 |
| 2 | 80 | 16 |
| 3 | 160 | 32 |
| 4 | 200 | 40 |
| 5 | 400 | 80 |
| 6 | 400 | 80 |
| 7 | 400 | 80 |
| " | " | " |
| " | " | " |
| 26 | 400 | 80 |

EXAMPLE 4

Penicillin Hypersensitive Patient Treated with Penicillin-Antibody Complexes

Anaphylactic reactions to medications like penicillin or sulfamides are treated as follows:

A patient suffering from allergy to penicillin is treated according to the following procedure. Benzyl-penicilloyl (BPO) moities are coupled to a carrier protein, viz. human serum albumin (HSA). The BPO-HSA complex is then coupled to a Sepharose solid phase by a standard cyanogen bromide activation process. Such process is described in "Affinity Chromatography" by M. Wilchek, T. Miron and J. Kohn in Methods in Enzymology, Vol. 104, pages 3-55, edited by W. B. Jakoby and published 1984 by Academic Press, Inc.

The patient receives intradermally BPO-coupled human serum albumin and his own penicillin-specific antibodies at a weight ratio of 1:30. The mixture is initially made of 300 ng of antibody and 10 ng of BPO-HSA allergen. The mixture is dissolved in 0.9% saline with 0.3% human serum albumin and 0.4% phenol as preservative. The amount of injected material is increased according to the scheme listed in Table 6 up to a maxiumum dose of 3000 ng of antibody and 100 ng of allergen per inoculation.

No systemic side effect is noted. At the end of the treatment significant reduction of the hypersensitivity to penicillin is noted.

TABLE 6

| Penicillin Hypersensitive Patient Injection Scheme | | |
|---|---|---|
| Injection | Antibody (ng) | Allergen (ng) |
| 1 | 300 | 10 |
| 2 | 600 | 20 |
| 3 | 1200 | 40 |
| 4 | 1800 | 60 |
| 5 | 2400 | 80 |
| 6 | 3000 | 100 |
| " | " | " |
| " | " | " |
| 15 | 3000 | 100 |

EP 0 287 361 B1

EXAMPLE 5

Use of Synthetic and Genetically Engineered Peptides as Allergen in Treatment of DPT Hypersensitivity

Most allergens carry a small number of antigenic determinants which are made of a small number of amino acids, usually 3 to 6. These antigenic determinants are synthesized in vitro and used instead of the original antigen. They are usually coupled to a carrier protein with variable degrees of substitution. As an alternative, the antigenic determinants are purified and sequenced and the corresponding DNA sequence determined. This DNA is introduced in the genome of a microorganism which is used to synthesize the allergen in vitro. References teaching suitable genetic engineering tech niques include (1) Molecular Cloning, by T. Maniatis, E.F. Fritsch, and J. Sambrook, published 1982 by Cold Spring Harbor Laboratory, (2) Solid Phase Biochemistry, by B.R. Wallace and K. Itakura, page 631, edited by W.H. Scouten and published 1983 by J. Wiley & Sons, New York, and (3) K. Murray, Philosophical Transactions of the Royal Society of London - Part B, Vol. 290, pages 369-386 (1980).

A patient hypersensitive to P1, a major allergen of D.pteronyssinus (see Examples 2 and 3) is treated by regular intradermal injections of autologous anti-P1 specific antibodies mixed with a synthetic peptide representative of the antigenic determinants of P1. The peptide was previously coupled to human serum albumin, with a degree of substitution of 5 (an average of 5 peptides per albumin molecule). The weight ratio of allergen to antibody is 1:10. The injections are made weekly, starting at an allergen dosage of 10 ng and followed by two-fold increases of the dosage to reach a dose of 100 $\mu$g allergen. After this treatment, no sign of hypersensitivity to P1 is detectable by skin testing or a bronchial provocation test, and the level of specific anti-P1 IgE and IgG is decreased.

Having illustrated the practice of the invention in connection with several specific embodiments, those of ordinary skill in the art are taught to vary the elements of the invention, such as allergen dosage, allergen-antibody ratio, carrier, preservative, preparation of allergen-specific antibodies, and manner and periodicity of administration as may be appropriate to treat immediate hypersensitivity that is mediated by IgE antibody. The examples are modified in alternative embodiments by deriving the antibodies from plasma pools, rather than individual patients. The examples are also modified in alternative embodiments by the preparation of various types of insolubilized allergen supports.

**Claims**

1.  A pharmaceutical composition for administration to human beings, for the treatment of immediate hypersensitivity to an allergen, said composition comprising an immune complex of an allergen and a purified antibody specific thereto, said allergen being selected from a specific subclass of antigen that can cause immediate hypersensitivity that is mediated by IgE antibody, said allergen also comprising an in vitro synthesised antigenic determinant coupled to a carrier protein; said antibody being present in at least a molar equivalent of antigenic determinant; and a pharmacologically acceptable carrier or diluent.

2.  A pharmaceutical composition for administration to human beings, for the treatment of immediate hypersensitivity to an allergen, said composition comprising an immune complex of an allergen and a purified antibody specific thereto, said allergen being selected from a specific subclass of antigen that can cause immediate hypersensitivity that is mediated by IgE antibody and said allergen also being chemically modified by glutaraldehyde or ethylene glycol derivatives; said antibody being present in at least a molar equivalent of the allergenic determinants of said allergen; and a pharmacologically acceptable carrier or diluent.

3.  The composition of Claim 1 or 2 wherein the amount of antibody is such that, when the composition is administered, there is no significant allergenic effect.

4.  The composition of any preceding Claim wherein the antibody is present, relative to antigenic determinant, in a molar excess.

5.  The composition of Claim 1 or 4, wherein the antigenic determinant to antibody molar ratio is from about 1:1 to about 1:500.

14

6. The composition of any one of the preceding claims wherein the allergen is present in a dose of at least about 1 ng.

7. The composition of any one of the preceding claims wherein said carrier is a liquid and said composition is in sterile injectable form.

8. The composition of Claim 7 wherein the liquid is saline.

9. The composition of any one of Claims 1 to 8 wherein said composition is in the form of a slow-release implant, a spray, an aerosol, drops or oral dose.

10. The composition of any one of Claims 1 to 8 in lyophilized form.

11. The composition of any one of the preceding claims wherein said antibody is derived from a patient suffering immediate hypersensitivity to the allergen.

12. The composition of any one of Claims 1 to 10 wherein said antibody is derived from pooled plasma from multiple donors.

13. The composition of any one of the preceding claims wherein said antibody is a polyclonal antibody.

14. The composition of any one of Claims 1 to 10 wherein said antibody is a mixture selected from the group consisting of several monoclonal antibodies and fractions derived from several monoclonal antibodies.

15. The composition of Claim 13 wherein said antibody is in the form of $F(ab')_2$ fragments.

16. The composition of any one of the preceding claims wherein said composition comprises two or more allergens and two or more respective families of antibodies thereto.

17. The use of a mixture of an allergen and an antibody specific thereto in the manufacture of a medicament for reducing the immediate hypersensitivity of a human being to the allergen, said allergen being selected from a specific subclass of antigen which can cause immediate hypersensitivity which is mediated by IgE antibody and said allergen also comprising an in vitro synthesized antigenic determinant coupled to a carrier protein, said antibody being present in at least a molar equivalent of antigenic determinant.

18. The use of a mixture of an allergen and an antibody specific thereto in the manufacture of a medicament for reducing the immediate hypersensitivity of a human being to the allergen, said allergen being selected from a specific subclass of antigen which can cause immediate hypersensitivity which is mediated by IgE antibody and said allergen also being chemically modified by glutaraldehyde or ethylene glycol derivatives, and wherein said antibody is present in at least molar equivalent of the allergenic determinants of said allergen.

19. The use of the mixture of any one of Claims 17 and 18 wherein the antibody is derived from said human being before said mixture is formed.

20. The use of the mixture of any one of Claims 17 to 19 wherein said antibody is derived from a pooled plasma from multiple donors before said mixture is formed.

21. The use of the mixture of any one of Claims 17 to 20 in the manufacture of an injectable medicament.

**Patentansprüche**

1. Pharmazeutische Zusammensetzung zur Verabreichung an Menschen zur Behandlung von Hypersensitivität vom Soforttyp auf ein Allergen, wobei die Zusammensetzung folgendes aufweist: einen Immunkomplex eines Allergens und eines dafür spezifischen gereinigten Antikörpers, wobei das Allergen aus einer spezifischen Antigen-Unterklasse ausgewählt ist, die Hypersensitivität vom Soforttyp verursachen

EP 0 287 361 B1

kann, die durch IgE-Antikörper vermittelt wird, wobei das Allergen außerdem eine *in vitro* synthetisierte Antigen-Determinante aufweist, die an ein Transportprotein gekoppelt ist; wobei der Antikörper in mindestens einem molaren Äquivalent der Antigen-Determinante anwesend ist; sowie

einen pharmakologisch akzeptablen Träger oder ein solches Verdünnungsmittel.

2. Pharmazeutische Zusammensetzung zur Verabreichung an Menschen zur Behandlung von Hypersensitivität vom Soforttyp auf ein Allergen, wobei die Zusammensetzung folgendes aufweist: einen Immunkomplex eines Allergens und eines dafür spezifischen gereinigten Antikörpers, wobei das Allergen aus einer spezifischen Antigen-Unterklasse ausgewählt ist, die Hypersensitivität vom Soforttyp verursachen kann, die durch IgE-Antikörper vermittelt wird, und wobei das Allergen außerdem durch Glutaraldehyd- oder Ethylenglykol-Derivate chemisch modifiziert ist; wobei der Antikörper in mindestens einem molaren Äquivalent der Allergen-Determinanten des Allergens anwesend ist; sowie

einen pharmakologisch akzeptablen Träger oder ein solches Verdünnungsmittel.

3. Zusammensetzung nach Anspruch 1 oder 2,
wobei die Menge an Antikörper derart ist, daß dann, wenn die Zusammensetzung verabreicht wird, keine signifikante allergene Wirkung auftritt.

4. Zusammensetzung nach einem der vorhergehenden Ansprüche,
wobei der Antikörper relativ zu der Antigen-Determinanten in einem molaren Überschuß anwesend ist.

5. Zusammensetzung nach Anspruch 1 oder 4,
wobei das Molverhältnis von Antigen-Determinante zu Antikörper zwischen ca. 1:1 und ca. 1:500 liegt.

6. Zusammensetzung nach einem der vorhergehenden Ansprüche,
wobei das Allergen in einer Dosis von mindestens ca. 1 ng anwesend ist.

7. Zusammensetzung nach einem der vorhergehenden Ansprüche,
wobei der Träger eine Flüssigkeit ist und die Zusammensetzung in steriler injizierbarer Form vorliegt.

8. Zusammensetzung nach Anspruch 7,
wobei die Flüssigkeit Kochsalzlösung ist.

9. Zusammensetzung nach einem der Ansprüche 1 bis 8,
wobei die Zusammensetzung in Form eines langsam freisetzenden Implantats, eines Sprays, eines Aerosols, von Tropfen oder einer Oraldosis vorliegt.

10. Zusammensetzung nach einem der Ansprüche 1 bis 8 in lyophilisierter Form.

11. Zusammensetzung nach einem der vorhergehenden Ansprüche,
wobei der Antikörper von einem Patienten gewonnen ist, der an Hypersensitivität vom Soforttyp auf das Allergen leidet.

12. Zusammensetzung nach einem der Ansprüche 1 bis 10,
wobei der Antikörper aus Poolplasma von einer Vielzahl von Spendern gewonnen ist.

13. Zusammensetzung nach einem der vorhergehenden Ansprüche,
wobei der Antikörper ein polyklonaler Antikörper ist.

14. Zusammensetzung nach einem der Ansprüche 1 bis 10,
wobei der Antikörper ein Gemisch ist, das aus der Gruppe ausgewählt ist, die aus mehreren monoklonalen Antikörpern und Fraktionen, die von mehreren monoklonalen Antikörpern gewonnen sind, besteht.

15. Zusammensetzung nach Anspruch 13,
wobei der Antikörper in Form von F(ab')$_2$-Fragmenten vorliegt.

16

16. Zusammensetzung nach einem der vorhergehenden Ansprüche,
wobei die Zusammensetzung zwei oder mehr Allergene und zwei oder mehr entsprechende Familien von Antikörpern dafür aufweist.

17. Verwendung eines Gemischs von einem Allergen und einem dafür spezifischen Antikörper bei der Herstellung eines Medikaments, um die Soforttyp-Hypersensitivität eines Menschen auf das Allergen zu verringern, wobei das Allergen aus einer spezifischen Antigen-Unterklasse ausgewählt ist, die Hypersensitivität vom Soforttyp verursachen kann, die durch IgE-Antikörper vermittelt wird, und wobei das Allergen außerdem eine *in vitro* synthetisierte Antigen-Determinante aufweist, die an ein Transportprotein gekoppelt ist, wobei der Antikörper in mindestens einem molaren Äquivalent der Antigen-Determinante anwesend ist.

18. Verwendung eines Gemischs von einem Allergen und einem dafür spezifischen Antikörper bei der Herstellung eines Medikaments, um die Soforttyp-Hypersensitivität eines Menschen auf das Allergen zu verringern, wobei das Allergen aus einer spezifischen Antigen-Unterklasse ausgewählt ist, die Hypersensitivität vom Soforttyp verursachen kann, die durch IgE-Antikörper vermittelt wird, und wobei das Allergen außerdem durch Glutaraldehyd- oder Ethylenglykol-Derivate chemisch modifiziert ist, und wobei der Antikörper in mindestens einem molaren Äquivalent der Allergen-Determinanten des Allergens anwesend ist.

19. Verwendung des Gemischs nach einem der Ansprüche 17 und 18, wobei der Antikörper von dem Menschen gewonnen wird, bevor das Gemisch gebildet wird.

20. Verwendung des Gemischs nach einem der Ansprüche 17 bis 19, wobei der Antikörper aus einem Poolplasma von einer Vielzahl von Spendern gewonnen wird, bevor das Gemisch gebildet wird.

21. Verwendung des Gemischs nach einem der Ansprüche 17 bis 20 bei der Herstellung eines injizierbaren Medikaments.

**Revendications**

1. Préparation pharmaceutique à usage humain pour le traitement de l'hypersensibilité immédiate à un allergène, ladite préparation comprenant un complexe immun constitué d'un allergène et d'un anticorps spécifique purifié, ledit allergène étant sélectionné dans une sous-classe spécifique d'antigène qui provoque une hypersensibilité immédiate par l'intermédiaire d'un anticorps IgE, ledit allergène comprenant également un déterminant antigénique synthétisé in vitro, couplé à une protéine vectrice; ledit anticorps étant présent dans au moins un équivalent molaire de déterminant antigénique; et un vecteur ou un diluant pharmacologiquement acceptables.

2. Préparation pharmaceutique à usage humain pour le traitement de l'hypersensibilité immédiate à un allergène, ladite préparation comprenant un complexe immun constitué d'un allergène et d'un anticorps spécifique purifié, ledit allergène étant sélectionné dans une sous-classe spécifique d'antigène qui provoque une hypersensibilité immédiate par l'intermédiaire d'un anticorps IgE, ledit allergène étant également modifié chimiquement par des dérivés de glutaraldéhyde ou d'éthylène glycol; ledit anticorps étant présent dans au moins un équivalent molaire des déterminants allergéniques dudit allergène; et un vecteur ou un diluant pharmacologiquement acceptables.

3. Préparation selon la revendication 1 ou 2 dans laquelle la quantité d'anticorps est telle que l'administration de la préparation n'entraîne aucun effet allergénique significatif.

4. Préparation selon l'une quelconque des revendications précédentes, dans laquelle l'anticorps est présent, par rapport au déterminant antigénique, en excédent molaire.

5. Préparation selon la revendication 1 ou 4 dans laquelle le rapport molaire déterminant antigénique-anticorps est compris entre 1:1 environ et 1:500 environ.

6. Préparation selon l'une quelconque des revendications précédentes dans laquelle l'allergène est présent à une dose d'au moins 1 ng.

**7.** Préparation selon l'une quelconque des revendications précédentes dans laquelle le vecteur est un liquide et ladite préparation une forme injectable stérile.

**8.** Préparation selon la revendication 7 dans laquelle le liquide est une solution saline.

**9.** Préparation selon l'une quelconque des revendications 1 à 8 dans laquelle ladite préparation est sous forme d'un implant à libération retard, d'un spray, d'un aérosol, de gouttes ou d'une dose orale.

**10.** Préparation selon l'une quelconque des revendications 1 à 8, sous forme lyophilisée.

**11.** Préparation selon l'une quelconque des revendications précédentes dans laquelle ledit anticorps provient d'un patient souffrant d'hypersensibilité immédiate à l'allergène.

**12.** Préparation selon l'une quelconque des revendications 1 à 10 dans laquelle ledit anticorps provient d'un pool de plasma de donneurs multiples.

**13.** Préparation selon l'une quelconque des revendications précédentes dans laquelle ledit anticorps est un anticorps polyclonal.

**14.** Préparation selon l'une quelconque des revendications 1 à 10 dans laquelle ledit anticorps est un mélange sélectionné à partir d'un groupe consistant en plusieurs anticorps monoclonaux et fractions dérivées de plusieurs anticorps monoclonaux.

**15.** Préparation selon la revendication 13 dans laquelle ledit anticorps est sous forme de fragments F(ab')2.

**16.** Préparation selon l'une quelconque des revendications précédentes dans laquelle ladite préparation comprend deux allergènes ou plus, et deux familles respectives d'anticorps ou plus.

**17.** Utilisation d'un mélange d'allergène et d'anticorps spécifique pour la fabrication d'un médicament pour réduire l'hypersensibilité immédiate d'un être humain à l'allergène, ledit allergène étant sélectionné dans une sous-classe spécifique d'antigène qui provoque une hypersensibilité immédiate par l'intermédiaire d'un anticorps IgE, ledit allergène comprenant également un déterminant antigénique synthétisé in vitro, couplé à une protéine vectrice; ledit anticorps étant présent dans au moins un équivalent molaire de déterminant antigénique; et un vecteur ou un diluant pharmacologiquement acceptables.

**18.** Utilisation d'un mélange d'allergène et d'anticorps spécifique dans la fabrication d'un médicament pour réduire l'hypersensibilité immédiate d'un être humain à l'allergène, ledit allergène étant sélectionné dans une sous-classe spécifique d'antigène qui provoque une hypersensibilité immédiate par l'intermédiaire d'un anticorps IgE, ledit allergène étant également modifié chimiquement par des dérivés de glutaraldéhyde ou d'éthylène glycol; ledit anticorps étant présent dans au moins un équivalent molaire des déterminants allergéniques dudit allergène.

**19.** Utilisation du mélange de l'une quelconque des revendications 17 et 18, dans lequel l'anticorps provient dudit humain avant la formation du mélange.

**20.** Utilisation du mélange de l'une quelconque des revendications 17 à 19 dans lequel ledit anticorps provient d'un pool de plasma de donneurs multiples, avant la formation du mélange.

**21.** Utilisation du mélange de l'une quelconque des revendications 17 à 20 pour la fabrication d'un médicament injectable.